(19) Europäisches Patentamt
European Patent Office
Office européen des brevets

(11) Veröffentlichungsnummer: **0 271 761 B1**

(12) **EUROPÄISCHE PATENTSCHRIFT**

(45) Veröffentlichungstag der Patentschrift: **18.03.92**

(51) Int. Cl.⁵: **C07D 275/02**

(21) Anmeldenummer: **87117751.5**

(22) Anmeldetag: **01.12.87**

# (54) Verfahren zur Isolierung von Isothiazolinonderivaten.

(30) Priorität: **18.12.86 DE 3643183**

(43) Veröffentlichungstag der Anmeldung:
**22.06.88 Patentblatt 88/25**

(45) Bekanntmachung des Hinweises auf die Patenterteilung:
**18.03.92 Patentblatt 92/12**

(84) Benannte Vertragsstaaten:
**DE ES FR GB IT**

(56) Entgegenhaltungen:
**EP-A- 0 095 907**

**CHEMICAL ABSTRACTS, Band 70, Nr. 11, 17. März 1969, Columbus, Ohio, US; CHAN, A.W.K.; CROW, W.D.: "Isothiazole chemistry. V. Acylation and acyl migration in 3-hydroxy isothiazole." Seite 326, Spalte 1, Zusammenfassung-Nr. 47 354d**

**CHEMICAL ABSTRACTS, Band 87, Nr. 19, 7. November 1977, Columbus, Ohio, US; WEILER, E.D.; PETIGARA, R.B.; WOLFERSBERGER, M.H.; MILLER, G.A.: "Isothiazoles. IX. Halogenation of 2-substituted-4-isothiazolin-3-ones" Seite 582, Spalte 1, Zusammenfassung-Nr. 152 065j**

**CHEMICAL ABSTRACTS, Band 73, Nr. 7, 17. August 1970, Columbus, Ohio, US; CHAN, A.W.K.; CROW, W.D.; GOSNEY, I.: "Isothiazole chemistry. X. Acylation, alkylation and tantomerism in 3-hydroxy isothiazole." Seite 320, Spalte 2, Zusammenfassung-Nr. 35 262w**

(73) Patentinhaber: **RIEDEL-DE HAEN AKTIENGESELLSCHAFT**
**Wunstorfer Strasse 40**
**W-3016 Seelze 1(DE)**

(72) Erfinder: **Lindner, Wolfgang, Dr.**
**Leinestrasse 3**
**W-3016 Seelze 1(DE)**

(74) Vertreter: **Urbach, Hans-Georg, Dr. et al**
**Hanauer Landstrasse 526**
**W-6000 Frankfurt am Main 61(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 271 761 B1

**Beschreibung**

Die vorliegende Erfindung betrifft ein Verfahren zur Isolierung von Isothiazolinonderivaten der Formel I

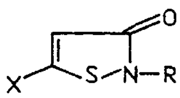

(I)

worin

entweder R Alkyl mit 1 bis 3 C-Atomen und X Wasserstoff oder Halogen
oder R Alkyl mit 1 bis 16 C-Atomen oder Cycloalkyl mit 5 oder 6 C-Atomen und X Wasserstoff bedeutet,

aus technischen Gemischen, die von der Herstellung her noch unerwünschte Begleitstoffe enthalten.

Die Verbindungen der Formel I, in denen R und X die oben angegebenen Bedeutungen haben, sind wertvolle Mikrobiozide mit einem breiten Wirkungsspektrum gegen Bakterien, Hefen, Pilze und Algen (N.S. Lewis, G.A. Miller; FR 1.555 415, 24.1.1969). Sie werden besonders häufig in der Praxis zum Schutz technischer Materialien sowie kosmetischer Emulsionen vor Mikrobenbefall eingesetzt.

Zum Beispiel wird eine Formulierung einer Mischung aus 2-Methyl-isothiazolin-3-on und 5-Chlor-2-methyl-isothiazol-3-on zum Schutz von technischen Dispersionen sowie kosmetischer Emulsionen vor Mikrobenbefall in der Praxis verwendet. Das 2-n-Octyl-isothiazolin-3-on findet Anwendung als Fungizid in technischen Materialien, wie zum Beispiel Leder, Fassadenbeschichtungen und Kunststoffen.

Die Herstellung der 2-Alkyl-isothiazolin-3-one ist aus der Literatur bekannt. (S.N. Lewis, G.A. Miller, M. Hausmann, E.C. Szamborski; J. Heterocycl. Chem. 8, S. 571ff (1971)).

Man cyclisiert $\beta,\beta'$-Dithio-bis-(propionsäure-N-alkylamid) durch Reaktion mit 3 Äquivalenten Halogen, vorzugsweise Chlor. Die dabei als Zwischenprodukt auftretenden entsprechenden Sulfensäurechloride können dabei vor ihrer Cyclisierung weiter chloriert werden und ergeben dann in Position 5 chlorierte 2-Alkyl-isothiazolin-3-one. Auch die Endprodukte besitzen eine Reaktivität mit Chlor, und es entstehen auf diesem Wege im Reaktionsgemisch 4-Halogen-2-alkyl-isothiazolin-3-one, 4,5-Dichlor-2-alkyl-isothiazolin-3-one, sowie des weiteren deren Halogenadditionsverbindungen, nämlich tri-bzw. tetrachlorierte 2-Alkyl-isothiazolidin-3-one der folgenden Struktur II:

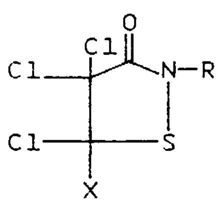

(II)

wobei X und R die oben bei Struktur I angegebenen Substituenten repräsentieren.

Ferner können die technischen Produkte noch wasserlösliche Substanzen wie $\beta$-Alkylamino-propionsäure-N'-alkylamid, Alkylamine und ggf. auch deren Nitrosoverbindungen enthalten.

Die rohen technischen Produkte enthalten somit neben den Zielprodukten der obigen Formel I noch Verbindungen der Formel I, in denen R Alkyl mit 4 bis 16 C-Atomen oder Cycloalkyl mit 5 oder 6 C-Atomen und X Halogen bedeutet, und/oder 4-Halogen-2-alkyl-isothiazolin-3-one und/oder 4,5-Dihalogen-2-alkyl-isothiazolin-3-one und/oder deren Halogenadditionsverbindungen und/oder Nitrosoverbindungen und/oder, ggf., wasserlösliche Amine und Amide.

Obgleich auch die höher chlorierten Produkte biologische Aktivität gegenüber Mikroorganismen aufweisen, ist ihre Anwesenheit, besonders die der polychlorierten Isothiazolinone, in dem technischen Biozid häufig nicht erwünscht. Aber auch die Anwendung der Verbindungen der Formel I als Fungizide für Anstrichfarben erfordert es, wegen der Inhalationstoxizität der höher chlorierten Isothiazolinonderivate, diese und andere Verunreinigungen aus dem technischen Produkt zu entfernen. Technische 2-n-Octyl-isothiazolin-3-on-Formulierungen können nicht zur Fungizidausrüstung von PVC-Weichmachern verwendet werden, da die Verunreinigungen bei der hohen Verarbeitungstemperatur zu einer Zersetzung des Wirkstoffes und zu einer Vergilbung des Endproduktes führen.

Verbindungen der Formel I, die einen längerkettigen Alkylrest oder einen Cycloalkylrest aufweisen, sollen auch keine monochlorierten Derivate enthalten.

Ein weiteres Problem bei der Anwendung einer mit Magnesiumnitrat stabilisierten wäßrigen Formulierung von 2-Methyl-isothiazolinon-3 und 5-Chlor-2-methyl-isothiazolinon-3 zur Konservierung kosmetischer Mittel stellt die Gefahr der Bildung von Nitrosaminen aus in der Mischung als Verunreinigung vorkommenden sekundären Aminen dar. Nitrosamine können auch von vornherein in den technischen Lösungen der 2-Methyl-isothiazolinonderivate vorliegen. (Vergl. EP-A-0095907).

Es besteht daher ein dringendes Bedürfnis nach Verfahren, welche eine Isolierung der gewünschten, wertvollen Substanzen, nämlich 2-Niederalkyl-isothiazolin-3-one, 5-Monochlor-2-niederalkyl-isothiazolin-3-one und 2-Höheralkyl-isothiazolin-3-one, wie sie durch die Formel I definiert sind, aus den mit den oben genannten, vielfältigen Begleitstoffen verunreinigten technischen Produkten gestatten.

Es wurde nun überraschenderweise gefunden, daß es gelingt, selektiv die verschiedenen, gewünschten Verbindungen der Formel I von den zahlreichen genannten, unerwünschten Begleitstoffen zu trennen und zu isolieren.

Isolieren im Sinne der folgenden Beschreibung bedeutet nicht nur die Gewinnung einer einheitlichen Verbindung in fester Form, sondern es soll auch die Gewinnung einer Lösung umfassen, die eine solche einheitliche Verbindung enthält. Ferner soll unter Isolierung der Verbindungen der Formel I auch die Gewinnung eines Gemisches von Verbindungen der Formel I verstanden werden, das jedoch frei ist von den störenden Begleitstoffen, oder die Gewinnung einer Lösung eines solchen Gemisches von Verbindungen der Formel I.

Das erfindungsgemäße Verfahren arbeitet überraschend einfach und ist lediglich leicht zu modifizieren, je nachdem, ob das technische Gemisch wasserlösliche Verunreinigungen enthält oder nicht.

Bei Abwesenheit wasserlöslicher Verunreinigungen in dem technischen Rohprodukt, aus dem die Verbindungen der Formel I isoliert werden sollen, wird erfindungsgemäß so verfahren, daß man das technische Gemisch in einem mit Wasser nicht oder wenig mischbaren organischen Lösungsmittel auflöst, die Lösung trocknet, anschließend mit einer starken Säure versetzt und die ausfallenden Salze der Verbindung der Formel I abtrennt und gewünschtenfalls hydrolysiert.

Enthält das technische Rohprodukt, aus dem die Verbindungen der Formel I isoliert werden sollen, wasserlösliche Verunreinigungen, so wird erfindungsgemäß so verfahren, daß man das technische Gemisch zunächst mit einem Zweiphasensystem, bestehend aus einem mit Wasser nicht oder wenig mischbaren organischen Lösungsmittel und Wasser oder einer verdünnten Säure behandelt, die Phasen voneinander trennt, die organische Phase trocknet und anschließend mit einer starken Säure versetzt und die aus der organischen Phase ausfallenden Salze der Verbindung der Formel I abtrennt und gewünschtenfalls hydrolysiert.

Selbstverständlich können nach dieser Verfahrensvariante auch solche technischen Gemische verarbeitet werden, die keine störenden wasserlöslichen Begleitstoffe enthalten.

Auch wenn das technische Rohprodukt nicht in fester Form sondern als wäßrige Lösung oder Suspension vorliegt, ist es zweckmäßig, quasi nach der zweiten Verfahrensvariante zu verfahren. Es ist dann nicht erforderlich, das technische Rohprodukt in fester Form zu isolieren, sondern die wäßrige Lösung bzw. Suspension wird mit dem wasserunlöslichen organischen Lösungsmittel versetzt, intensiv gemischt und dieses Zweiphasensystem direkt wie oben beschrieben weiterbehandelt.

Beim Arbeiten nach der letzgenannten Verfahrensvariante finden sich Verbindungen der Formel I, in denen R Alkyl mit 1 bis 3 C-Atomen und X Wasserstoff bedeuten, praktisch vollständig und solche, in denen R Alkyl mit 1 bis 3 C-Atomen und X Chlor bedeuten, zum Teil in der wäßrigen Phase des Zweiphasensystems. Sofern man auf die Isolierung dieser Verbindungen Wert legt und diese wäßrige Phase keine störenden Begleitstoffe enthält, ist die wäßrige Phase als solche oder die darin gelöste Verbindung der Formel I, ggf. gemeinsam mit den aus der organischen Phase abgetrennten Verbindungen der Formel I bzw. deren Salzen, der Weiterverarbeitung, z.B. der Formulierung zu einer anwendungsfertigen Zubereitung, zuzuführen.

Sehr zweckmäßig ist es in diesem Fall, die wäßrige Phase zur Hydrolyse der aus der organischen Phase durch die starke Säure ausgefällten Salze der Verbindungen der Formel I einzusetzen.

Organische Lösungsmittel, die für die Durchführung des erfindungsgemäßen Verfahrens in Betracht kommen, sind solche, die sich möglichst wenig oder gar nicht in Wasser lösen, die ihrerseits möglichst wenig oder gar kein Wasser aufnehmen, und ein gutes Lösungsvermögen für die Verbindungen der Formel I aufweisen.

Vorteilhafterweise wählt man hierbei solche organische Lösungsmittel aus, deren Dichte möglichst weit von 1 abweicht, damit eine möglichst problemlose Phasentrennung erfolgt.

Vorzugsweise verwendet man solche Lösungsmittel, deren eigene Basizität mindestens eine Zehnerpotenz geringer ist als die Basizität der Verbindungen der Formel I. Ferner ist es bevorzugt, aprotische organische Lösungsmittel einzusetzen.

Schließlich ist es vorteilhaft, organische Lösungsmittel mit einem nicht zu hohen Siedepunkt einzusetzen, weil diese Maßnahme das Trocknen der ausgefällten und abgetrennten Salze der Verbindungen der Formel I erleichtert.

Organische Lösungsmittel, die den obigen Anforderungen entsprechen, sind z B. aliphatische Halogenkohlenwasserstoffe, wie z .B. Methylenchlorid, Ethylenchlorid, Trichlorethan, Perchlorethylen; aromatische Kohlenwasserstoffe und Halogenkohlenwasserstoffe, wie z.B. Toluol, Xylol, technische Xylolgemische, Monochlorbenzol; Carbonsäureester, wie z.B. Ethylacetat, Propyl- und Isopropylacetat, Butylacetat; Dialkylether, wie z.B. Diethylether, Dipropylether, Dibutylether, Ethylbutylether.

Selbstverständlich können auch Gemische der obigen Lösungsmittel, sofern sie die übrigen, oben genannten Spezifikationen der für das erfindungsgemäße Verfahren brauchbaren organischen Lösungsmittel erfüllen, insbesondere die Dichtebedingung erfüllen, für die Durchführung des erfindungsgemäßen Verfahrens eingesetzt werden.

Bevorzugt wird als organisches Lösungsmittel ein aliphatischer Halogenkohlenwasserstoff, ein Carbonsäureester oder ein Dialkylether oder eine Mischung solcher Lösungsmittel eingesetzt.

Bevorzugte Lösungsmittel für Isothiazolinone der Formel I mit kleinem 2-Alkylrest, wobei kleiner Alkylrest $C_1$-$C_4$-Alkyl bedeutet, sind chlorierte Kohlenwasserstoffe, wie beispielsweise Dichlormethan, Dichlorethan, u.a., Essigsäuremethylester, Essigsäureethylester, Diethylether, obwohl auch andere oder Gemische mit anderen Lösungsmitteln anzuwenden sind.

Bevorzugte Lösungsmittel für Isothiazolinone mit großem Alkylrest, wobei großer Alkylrest $C_5$-$C_{16}$-Alkyl bedeutet, sind Essigsäuremethylester, Essigsäureethylester, Diethylether, obwohl auch andere oder Gemische mit anderen Lösungsmitteln einzusetzen sind.

Bei der zweiten Verfahrensvariante erfolgt die Abtrennung der organischen Phase von der wäßrigen in bekannter Weise und mit technischen Geräten, die üblicherweise für die Trennung von Phasen unterschiedlicher Dichte verwendet werden, z.B. mittels Scheidetrichtern.

Das Trocknen der organischen Phase erfolgt ebenfalls in an sich bekannter Weise durch Zusatz eines Trockenmittels, wie z B. $Na_2SO_4$, oder Silikagel in ausreichender Menge, Rühren oder Schütteln der Mischung zur Beschleunigung des Trockenprozesses und anschließendes Abtrennen der getrockneten organischen Phase vom Trockenmittel, z.B. durch Filtrieren oder Zentrifugieren. Sofern das eingesetzte organische Lösungsmittel höher siedet als Wasser oder mit Wasser ein Azeotrop bildet, kann auch durch Andestillieren der organischen Phase ihr Wassergehalt erheblich erniedrigt oder ganz beseitigt werden.

Diese Operation kann zu einer erheblichen Einsparung von Trockenmitteln beitragen oder deren Verwendung vollständig erübrigen.

Aus der getrockneten organischen Phase werden die Verbindungen der Formel I durch starke Säuren als Salze gefällt. Die Fällung erfolgt nach der Gleichung

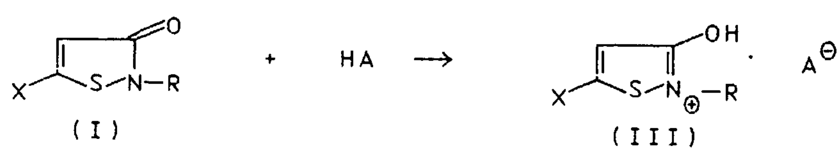


(I) (III)

worin HA die starke Säure und A$^\ominus$ demgemäß das Anion dieser starken Säure symbolisiert.

Die starke Säure kann selbstverständlich mehrbasisch sein.

Als starke Säuren zur Fällung der Isothiazoliumsalze der Formel III eignen sich alle Säuren mit pKA-Werten, die kleiner sind als 0, gemessen relativ zu Wasser (vergl. Cooksen in Chemical Reviews 74, S. 5-28 (1974)), wie z.B. Fluorsulfonsäure, Tetrafluoroborsäure, vorzugsweise wasserfreier Chlorwasserstoff (pKA = -7) und Bromwasserstoff (pKA = -9).

Wie aus den pKA-Wertangaben hervorgeht, scheiden wäßrige Säuren, wie z.B. Salzsäure, als Salzbildner für die 2-Alkylisothiazolin-3-one der Formel I aus.

Die aus der organischen Lösung gefällten Isothiazoliumsalze der Formel III werden wie üblich, z.B. durch Filtration oder Zentrifugieren, abgetrennt und, evtl. nach kurzem Nachwaschen mit reinem und möglichst niedrig siedendem organischen Lösungsmittel, getrocknet. Das Trocknen kann auch in manchen Fällen unterbleiben, wenn z.B. mit einem niedrig siedenden, wassermischbaren organischen Lösungsmittel nachgewaschen wurde.

Die gewünschtenfalls durchzuführende Hydrolyse der Salze der Formel III zu den Verbindungen der

Formel I kann einfach durch Umsetzung dieser Salze mit Wasser erfolgen.

Gegenüber Wasser verhalten sich die 2-Alkyl-3-hydroxy-isothiazoliumsalze der Struktur III nämlich als starke Säuren, so daß sie gemäß Gleichung I vollständig zu den entsprechenden wäßrigen Säuren hydrolysieren.

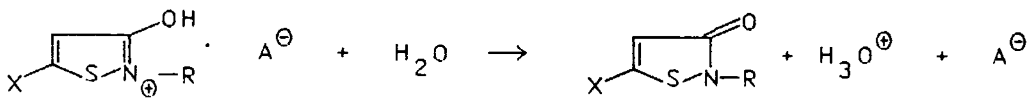

(III) (I)

Diese Reaktion kann in dem erfindungsgemäßen Verfahren in seiner letzten Stufe genutzt werden, um aus den Isothiazoliumsalzen der Formel III die gewünschten reinen Isothiazolin-3-one bzw. deren Lösungen herzustellen. Die Anwendung einer stärkeren Base als Wasser erübrigt sich dadurch.

Die Hydrolyse der Isothiazoliumsalze der Formel III kann demnach beispielsweise durch Eintragen in einen Überschuß Wasser erfolgen, wobei direkt wäßrige Lösungen oder Suspensionen der entsprechenden 2-Alkyl-isothiazolinone entstehen.

Ebensogut läßt sich die Hydrolse in einem organischen Lösungsmittel durchführen. Falls das eingesetzte organische Lösungsmittel nicht mit Wasser mischbar ist, kann die entstehende wäßrige Säure als separate Phase leicht abgetrennt werden, so daß eine Lösung des reinen 2-Alkylisothiazolinons in dem entsprechenden Lösungsmittel zur weiteren Anwendung anfällt.

Wie bereits oben beschrieben, kann man die Möglichkeit der Hydrolyse der Salze der Formel III durch Umsetzung mit Wasser auch zweckmäßig nutzen, um Verbindungen der Formel I, worin X Wasserstoff oder Chlor und R Alkyl mit 1 bis 3 C-Atomen bedeutet, aus einem technischen Gemisch, das keine störenden wasserlöslichen Begleitsubstanzen enthält, nach der zweiten Verfahrensvariante vollständig zu gewinnen. Man hat hierzu lediglich zur Hydrolyse der Salze der Verbindungen der Formel I die wäßrige Phase des Zweiphasensystems einzusetzen.

Das erfindungsgemäße Verfahren gestattet es, ausgehend von dem bei der technischen Cyclisierung von $\beta,\beta'$-Dithio-bis-(propionsäure-N-alkylamiden) anfallenden Vielstoffgemisch, auf einfache Weise gerade die Gruppe der interessierenden Substanzen der Formel I von der Gruppe der störenden Nebenprodukte zu trennen.

Beispiel 1

Das Beispiel veranschaulicht die Isolierung von 2-Methylisothiazolin-3-on und 5-Chlor-2-methylisothiazolin-3-on aus einer technischen Lösung, die als Hauptverunreinigung 4,5-Dichlor-2-methylisothiazolin-3-on enthält.

Ausgangsmaterial ist eine wäßrige technische Lösung mit einem Gehalt von 4,41 % 2-Methylisothiazolin3-on, 10,27 % 5-Chlor-2-methylisothiazolin-3-on und ca. 0,3 % 4,5-Dichlor-2-methyl-isothiazolin-3-on. Zur Abtrennung der Hauptverunreinigung werden 100 g der technischen Lösung mit 100 g Essigsäureethylester innig gemischt. Anschließend läßt man absitzen, trennt die organische Schicht in einem Scheidetrichter ab und trocknet sie über Natriumsulfat (Lösung B).

Die wäßrige Schicht (Lösung A) enthält nun
4,20 g 2-Methyl-isothiazolin-3-on (a)
5,73 g 5-Chlor-2-methyl-isothiazolin-3-on (b)
<0,01 g 4,5-Dichlor-2-methyl-isothiazolin-3-on (c),
die organische Phase (Lösung B)
0,21 g (a)
4,54 g (b)
0,3 g (c).

Die organische Lösung (B) wird nun mit Chlorwasserstoff gesättigt. Dabei werden 0,26 g 2-Methyl-3-hydroxy-isothiazoliumchlorid und 5,60 g 5-Chlor-2-methyl-3-hydroxy-isothiazoliumchlorid als leicht abfiltrierbare Feststoffe ausgefällt. Die farblosen Feststoffe wurden durch Filtration isoliert und in die wäßrige Lösung (A) eingetragen und unter Rühren aufgelöst.

Gemäß HPLC-Analyse hat die so erhaltene, gereinigte Formulierung folgende Zusammensetzung:
4,35 g (a)
9,96 g (b)

<0,01 g (c).

Beispiel 2

Das Beispiel veranschaulicht die Herstellung einer nitrosaminfreien 5-Chlor-2-methyl-isothiazolin-3-on-Lösung. 100 g einer wäßrigen technischen Lösung, die neben 2,9 % 2-Methyl-isothiazolin-3-on, 7,99 % 5-Chlor-2-methyl-isothiazolin-3-on, 0,21 % 4,5-Dichlor-2-methyl-isothiazolin-3-on noch 0,1 % β-(N-Nitroso-N-methylamino)-propionsäure-N′-methylamid enthält, werden analog der Beschreibung in Beispiel 1, zweimal mit je 100 g Dichlormethan extrahiert. Die organischen Extrakte werden dann über Natriumsulfat getrocknet und mit gasförmigem Chlorwasserstoff gesättigt. Der dabei erhaltene Feststoff wird durch Filtration isoliert und anschließend in 89 g Wasser aufgelöst. Man erhält so 100 g einer farblosen wäßrigen Lösung, deren Analyse in der folgenden Tabelle mit der der Ausgangslösung verglichen wird:

Tabelle 1

| Substanz | Menge in der Ausgangslösung | Menge in der gereinigten Lösung |
|---|---|---|
| 2-Methyl-isothiazolin-3-on | 2,90 g | 0,97 g |
| 5-Chlor-2-methyl-isothiazolin-3-on | 7,99 g | 7,75 g |
| 4,5-Dichlor-2-methyl-isothiazolin-3-on | 0,21 g | <0,01 g |
| β-(N-Nitroso-N-methylamino)-propionsäure-N'-methylamid | 0,1 g | nicht nachweisbar |

Zur Stabilisierung dieser Lösung wurde sie durch Zugabe von 257 g Magnesiumnitrat-hexahydrat und 357 g Wasser formuliert. Die Formulierung zeigte nach (14 Tagen bei 40 °C keinen Verlust an 5-chlor-2-methyl-isothiazolin-3-on.

Die so hergestellte Formulierung kann vorteilhaft zur Konservierung von kosmetischen Mitteln verwendet werden.

Beispiel 3:

a) Dieses Beispiel veranschaulicht die Abtrennung von 2-n-Octyl-isothiazolin-3-on aus einem Chlorierungsgemisch des β,β'-Dithio-bis-(propionsäure-N-octylamids).

7,0 g eines technischen Gemisches von 2-n-Octyl-isothiazolin-3-onen mit folgender HPLC-Analyse:
3,27 g 2-n-Octyl-isothiazolin-3-on
0,08 g 4-Chlor-2-n-octyl-isothiazolin-3-on
0,11 g 5-Chlor-2-n-octyl-isothiazolin-3-on
2,31 g 4,5-Dichlor-2-n-octyl-isothiazolin-3-on
0,02 g 4,4,5,5-Tetrachlor-2-n-octyl-isothiazolidin-3-on wurden in 20 g Essigsäureethylester gelöst und mit Chlorwasserstoffgas gesättigt. Der dabei entstehende Feststoff wurde abfiltriert und mit 5 g Essigsäureethylester gewaschen. Der Feststoff bestand aus reinem 2-n-Octyl-3-hydroxy-isothiazoliumchlorid.

Nach der HPLC-Analyse enthält das erhaltene Salz
2-n-Octyl-isothiazolin-3-on 3,14 g
4-Chlor-2-octyl-isothiazolin-3-on <0,01 g
5-Chlor-2-n-octyl-isothiazolin-3-on 0,01 g
4,5-Dichlor-2-n-octyl-isothiazolidin-3-on <0,01 g
4,4,5,5-Tetrachlor-2-n-octyl-isothiazolin-3-on <0,01 g

b) Herstellung einer reinen Formulierung von 2-n-Octyl-isothiazolin-3-on zur Fungizidausrüstung von Weich-PVC

100 g des nach obigem Beispiel hergestellten 2-n-Octyl-3-hydroxy-isothiazoliumchlorids (entsprechend 80,5 g 2-n-Octyl-isothiazolin-3-on) wurden mit 100 g Wasser hydrolysiert. Das freie 2-n-Octyl-isothiazolin-3-on wurde daraufhin mit 250 g eines Aromatengemisches vom Siedebereich 185-210 °C (®Shellsol AB) extrahiert. Die organische Phase wurde nach dem Absitzen abgetrennt, über Natriumsulfat getrocknet und in einen Weichmacher auf Basis eines Phthalsäure-di-höheralkylesters (®Imperon-Weichmacher) eingearbeitet.

Man erhält so 1000 g einer Formulierung (A), die zur Fungizidausrüstung von PVC eingesetzt werden kann. Sie enthält 8 % 2-n-Octyl-isothiazolin-3-on und weniger als 0,1 % chlorierte Isothiazolinone.

Zur Feststellung der Verwendbarkeit zur Fungizidausrüstung von Weich-PVC wurde die Formulierung (A) 30 Minuten auf 180° C erhitzt. Parallel dazu wurde eine analoge Formulierung (B), die aus technischem ungereinigtem 2-Octyl-isothiazolin-3-on gewonnen wurde, erhitzt.

| Ergebnis: | | |
|---|---|---|
| | Gehalt an 2-n-Octylisothiazolin-3-on | Aussehen |
| Formulierung (A) | 7,7 % | gelb - klar |
| Formulierung (B) | <0,1 % | tiefbraun - trüb |

<u>Beispiel 4:</u>

Das Beispiel veranschaulicht die Trennung eines Gemisches von 4-Brom-2-octyl-isothiazolin-3-on und 2-Octyl-isothiazolin-3-on

Eine Mischung von 10,0 g 4-Brom-2-octyl-isothiazolin-3-on (Reingehalt: 96,9 %) und 10,0 g 2-Octyl-isothiazolin-3-on (Reingehalt: 85,5 %) wurde in 50 ml Essigsäuremethylester gelöst. Die Lösung wurde in einem Eisbad abgekühlt und mit gasförmigem Bromwasserstoff gesättigt. Der dabei anfallende Feststoff wurde abfiltriert.

Auswaage: 11,8 g 2-Octyl-3-hydroxy-isothiazoliumbromid.

Das Salz wurde in 100 g 1,2-Dichlorethan aufgeschlämmt und durch Zugabe von 20 g Wasser hydrolysiert. Die organische Schicht wurde abgetrennt und im Vakuum eingedampft. Es wurde so 7,8 g 2-Octyl-isothiazolin-3-on isoliert, das nach HPLC einen Gehalt von <2 % des 4-Brom-Derivats hat.

**Patentansprüche**

**1.** Verfahren zur Isolierung von Isothiazolinonderivaten der Formel I

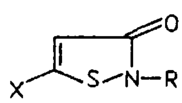

( I )

worin

entweder R Alkyl mit 1 bis 3 C-Atomen und X Wasserstoff oder Halogen

oder R Alkyl mit 1 bis 16 C-Atomen oder Cycloalkyl mit 5 oder 6 C-Atomen und X Wasserstoff bedeutet,

aus technischen Gemischen, die neben einer oder mehreren der zu isolierenden Verbindungen der Formel I noch Verbindungen der Formel I, in denen R Alkyl mit 4 bis 16 C-Atomen oder Cycloalkyl mit 5 oder 6 C-Atomen und X Halogen bedeutet, und/oder 4-Halogen-2-alkyl-isothiazolin-3-one und/oder 4,5-Dihalogen-2-alkyl-isothiazolin-3-one und/oder deren Halogenadditionsverbindungen und/oder Nitrosoverbindungen enthalten, dadurch gekennzeichnet, daß man die technischen Gemische in einem mit Wasser nicht oder wenig mischbaren organischen Lösungsmittel auflöst, die Lösung trocknet, anschließend mit einer starken Säure versetzt und die ausfallenden Salze der Verbindung der Formel I abtrennt und gewünschtenfalls hydrolysiert.

**2.** Verfahren zur Isolierung von Isothiazolinonderivaten der Formel I

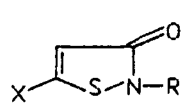

( I )

worin

entweder R  Alkyl mit 1 bis 3 C-Atomen und X Wasserstoff oder Halogen

oder R  Alkyl mit 1 bis 16 C-Atomen oder Cycloalkyl mit 5 oder 6 C-Atomen und X Wasserstoff bedeutet,

aus technischen Gemischen, die neben einer oder mehreren der zu isolierenden Verbindungen der Formel I noch Verbindungen der Formel I, in denen R Alkyl mit 4 bis 16 C-Atomen oder Cycloalkyl mit 5 oder 6 C-Atomen und X Halogen bedeutet, und/oder 4-Halogen-2-alkyl-isothiazolin-3-one und/oder 4,5-Dihalogen-2-alkyl-isothiazolin-3-one und/oder deren Halogenadditionsverbindungen und/oder Nitrosoverbindungen und/oder, ggf., wasserlösliche Amine und Amide enthalten, dadurch gekennzeichnet, daß man das technische Gemisch zunächst mit einem Zweiphasensystem, bestehend aus einem mit Wasser nicht oder wenig mischbaren organischen Lösungsmittel und Wasser oder einer verdünnten wäßrigen Säure behandelt, die Phasen voneinander trennt, die organische Phase trocknet und anschließend mit einer starken Säure versetzt und die aus der organischen Phase ausfallenden Salze der Verbindung der Formel I abtrennt und gewünschtenfalls hydrolysiert.

**3.** Verfahren gemäß Anspruch 1 oder 2, dadurch gekennzeichnet, daß das eingesetzte, mit Wasser nicht oder wenig mischbare organische Lösungsmittel bzw. die organische Phase des Zweiphasensystems ein aliphatischer Halogenkohlenwasserstoff, ein Carbonsäureester oder ein Dialkylether oder eine Mischung solcher Lösungsmittel ist.

**4.** Verfahren gemäß mindestens einem der Ansprüche 1 bis 3, dadurch gekennzeichnet, daß die eingesetzte starke Säure einen pKA-Wert hat, der kleiner als 0 ist

**5.** Verfahren gemäß mindestens einem der Ansprüche 1 bis 4, dadurch gekennzeichnet, daß zur Hydrolyse der Salze der Verbindungen der Formel I Wasser eingesetzt wird.

**6.** Verfahren gemäß mindestens einem der Ansprüche 2 bis 5, bei dem aus einem technischen Gemisch, das außer einer Verbindung der Formel I, worin R Alkyl mit 1 bis 3 C-Atomen und X Wasserstoff oder Chlor bedeutet, keine störenden wasserlöslichen Begleitsubstanzen enthält, zusätzlich diese Verbindungen der Formel I isoliert werden, dadurch gekennzeichnet, daß zur Hydrolyse der Salze der Verbindungen der Formel I die wäßrige Phase des Zweiphasensystems eingesetzt wird.

## Claims

**1.** Process for isolating isothiazolinone dervatives of the formula I

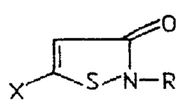

(I)

wherein

either R  denotes alkyl having 1 to 3 C atoms and X denotes hydrogen or halogen

or R  denotes alkyl having 1 to 16 C atoms or cycloalkyl having 5 or 6 C atoms and X denotes hydrogen,

from technical-grade mixtures which, in addition to one or more of the compounds of formula I to be isolated, contain compounds of formula I in which R denotes alkyl having 4 to 16 C atoms or cycloalkyl having 5 or 6 C atoms and X denotes halogen, and/or 4-halogen-2-alkylisothiazolin-3-ones and/or 4,5-dihalogen-2-alkyl-isothiazolin-3-ones and/or halogen addition compounds and/or nitroso compounds thereof, characterized in that the technical-glade mixtures are dissolved in an organic solvent that is immiscible or only slightly miscible in water, the solution is dried, subsequently a strong acid is added and the precipitating salts of formula I are separated and, if appropriate, hydrolized.

**2.** Process for isolating isothiazolinone derivatives of the formula I

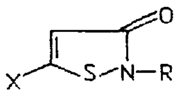

( I )

wherein

either R denotes alkyl having 1 to 3 C atoms and X denotes hydrogen or halogen

or R denotes alkyl having 1 to 16 C atoms or cycloalkyl having 5 or 6 C atoms and X denotes hydrogen,

from technical-grade mixtures which, in addition to one or more of the compounds of formula I to be isolated, contain compounds of formula I in which R denotes alkyl having 4 to 16 C atoms or cycloalkyl having 5 or 6 C atoms and X denotes halogen, and/or 4-halogen-2-alkylisothiazolin-3-ones and/or 4,5-dihalogen-2-alkyl-isothiazolin-3-ones and/or halogen addition compounds and/or nitroso compounds thereof, and/or, if appropriate, water-soluble amines and amides, characterized in that the technical-grade mixture is first treated with a two-phase mixture consisting of an organic solvent that is miscible or only slightly miscible with water and water or a dilute aqueous acid, the phases are separated from one another, the organic phase is dried, subsequently a strong acid is added and the salts of the compounds of formula I precipitating from the organic phase are separated and, if appropriate, hydrolized.

**3.** Process according to Claim 1 to 2, characterized in that the employed organic solvent that is immiscible or only slightly miscible with water or the organic phase of the two-phase system is an aliphatic halogen hydrocarbon, a carboxylic ester or a dialkyl ether or a mixture of such solvents.

**4.** Process according to at least one of the Claims 1 to 3, characterized in that the employed strong acid has a pKA value smaller than O.

**5.** Process according to at least one of the Claims 1 to 4, characterized in that water is used for hydrolizing the salts of the compounds of formula I.

**6.** Process according to at least one of the Claims 2 to 5, wherein from a technical-grade mixture which, except for a compound of formula I in which R denotes alkyl having to 1 to 3 C atoms and X denotes hydrogen or chlorine, does not contain detrimental water-soluble accompanying substances, there are additionally isolated these substances of formula I, characterized in that the aqueous phase of the two-phase system is employed for hydrolizing the salts of the compounds of formula I.

## Revendications

**1.** Procédé d'isolement de dérivés de l'isothiazolinone répondant à la formule I

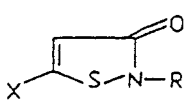

( I )

dans laquelle

soit R désigne un groupe alkyle en $C_1$ à $C_3$ et X un atome d'hydrogène ou d'halogène,

soit R désigne un groupe alkyle en $C_1$ à $C_{16}$ ou un groupe cycloalkyle en $C_5$ à $C_6$ et X un atome d'hydrogène,

à partir de mélanges techniques qui contiennent, en plus d'un ou plusieurs des composés répondant à la formule I à isoler, des composés répondant à la formule I dans lesquels R désigne un groupe alkyle en $C_4$ à $C_{16}$ ou cycloalkyle en $C_5$ à $C_6$ et X un atome d'halogène et/ou des 4-halogéno-2-alkyl-isothiazolin-3-ones et/ou des 4,5-dihalogéno-2-alkyl-isothiazolin-3-ones et/ou leurs composés d'addition aux halogènes et/ou des composés nitroso, caractérisé en ce qu'on dissout les mélanges techniques dans un solvant organique non miscible ou peu miscible à l'eau, en ce qu'on sèche la solution, puis en ce qu'on l'additionne d'un acide fort, en ce qu'on sépare les sels du composé répondant à la formule I qui précipitent et en ce qu'on les hydrolyse si on le désire.

**2.** Procédé d'isolement de dérivés de l'isothiazolinone répondant à la formule I

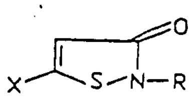

( I )

dans laquelle
soit R désigne un groupe alkyle en $C_1$ à $C_3$ et X un atome d'hydrogène ou d'halogène
soit R désigne un groupe alkyle en $C_1$ à $C_{16}$ ou un groupe cycloalkyle en $C_5$ à $C_6$ et X un atome d'hydrogène, à partir de mélanges techniques qui, outre un ou plusieurs des composés répondant à la formule I à isoler, contiennent également des composés répondant à la formule I dans lesquels R désigne un groupe alkyle en $C_4$ à $C_{16}$ ou un groupe cycloalkyle en $C_5$ à $C_6$ et X désigne un atome d'halogène, et/ou des 4-halogéno-2-alkyl-isothiazolin-3-ones et/ou des 4,5-dihalogéno-2-alkyl-isothiazolin-3-ones et/ou leurs composés d'addition aux halogènes et/ou leurs composés nitroso et/ou le cas échéant des amines et amides solubles dans l'eau, caractérisé en ce qu'on traite d'abord le mélange technique avec un système à deux phases constitué d'un solvant organique non miscible ou peu miscible à l'eau et d'eau ou d'un acide aqueux dilué, en ce qu'on sépare les phases l'une de l'autre, en ce qu'on sèche la phase organique puis en ce qu'on l'additionne d'un acide fort et en ce qu'on sépare les sels du composé répondant à la formule I qui précipitent dans la phase organique et en ce qu'on les hydrolyse si on le désire.

**3.** Procédé selon les revendications 1 ou 2, caractérisé en ce que le solvant organique utilisé, non miscible ou peu miscible à l'eau ou la phase organique du système à deux phases est un hydrocarbure halogéné aliphatique, un ester d'acide carboxylique ou un éther dialkylique ou un mélange de ces solvants.

**4.** Procédé selon au moins une des revendications 1 à 3, caractérisé en ce que l'acide fort utilisé a un pKA qui est inférieur à 0.

**5.** Procédé selon au moins une des revendications 1 à 4, caractérisé en ce qu'on utilise de l'eau pour l'hydrolyse des sels des composés répondant à la formule I.

**6.** Procédé selon au moins une des revendications 2 à 5, dans lequel, à partir d'un mélange technique qui ne contient, en dehors d'un composé répondant à la formule I dans lequel R désigne un groupe alkyle en $C_1$ à $C_3$ et X un atome d'hydrogène ou de chlore, aucune impureté soluble dans l'eau gênante, on isole en outre ces composés répondant à la formule I, caractérisé en ce qu'on utilise pour l'hydrolyse des sels des composés répondant à la formule I la phase aqueuse du système à deux phases.